# EUROPEAN PATENT SPECIFICATION

(11) **EP 2 219 546 B1**
(45) Date of publication and mention of the grant of the patent: **01.02.2017**
(21) Application number: 08855361.5
(22) Date of filing: 01.12.2008
(51) Int. Cl.: A61B 18/18, A61K 31/765, A61P 41/00, A61L 24/00, A61L 24/04

(54) **ENDOSCOPIC MUCOSAL RESECTIONING USING PURIFIED INVERSE THERMOSENSITIVE POLYMERS**
ENDOSKOPISCHE SCHLEIMHAUT-RESEKTIONIERUNG MIT GEREINIGTEN INVERSEN WÄRMEEMPFINDLICHEN POLYMEREN
RÉSECTION MUQUEUSE ENDOSCOPIQUE UTILISANT DES POLYMÈRES THERMOSENSIBLES INVERSES PURIFIÉS

(30) Priority: 29.11.2007 US 991049 P
(43) Date of publication of application: 25.08.2010
(73) Proprietor: Genzyme Corporation, Cambridge, MA 02142 (US)
(72) Inventor: VOGEL, Jean-Marie, Lincoln MA 01773 (US)
(74) Representative: Watson, Robert James
(86) International application number: PCT/US2008/085114
(87) International publication number: WO 2009/070793

(56) References cited:
- WO-A2-2005/046438
- US-A1- 2005 147 585
- US-A1- 2006 013 883
- US-A1- 2006 070 631
- CHUNG H J ET AL: "Synthesis and characterization of Pluronic<(>R) grafted chitosan copolymer as a novel injectable biomaterial", CURRENT APPLIED PHYSICS, NORTH-HOLLAND, AMSTERDAM, NL, vol. 5, no. 5, 1 July 2005 (2005-07-01), pages 485-488, XP027680596, ISSN: 1567-1739 [retrieved on 2005-07-01]
- ANNA S SCHENK ET AL: "Impurities in pluronic triblock copolymers can induce the formation of calcite mesocrystals", CHEMICAL GEOLOGY, ELSEVIER SCIENCE PUBLISHER B.V., AMSTERDAM, NL, vol. 294, 8 December 2011 (2011-12-08), pages 259-262, XP028452214, ISSN: 0009-2541, DOI: 10.1016/J.CHEMGEO.2011.12.007 [retrieved on 2011-12-16]

## Description

### Background of the Invention

Endoscopic mucosal resectioning (EMR) is acknowledged as a curative therapeutic modality in the treatment of early gastrointestinal cancers. Heretofore, submucosal injection with saline has been used to minimize complications during EMR. However, the duration of tissue elevation after saline injection is relatively short, and repeated injections are needed to perform EMR on large lesions. Notwithstanding the fact that solutions with high viscosity, such as hydroxypropyl methylcellulose (HPMC), have been used in EMR, an adequate solution to this problem has remained elusive.

US 2006/070631 discloses bulking or cushioning agents for use in medical procedures, including EMR.

### Summary of the Invention

One aspect of the invention relates to a composition comprising a purified inverse thermosensitive polymer for use in an endoscopic procedure for gastrointestinal mucosal resectioning in a mammal according to any one of claims 1 to 6, wherein said purified inverse thermosensitive polymer is poloxamer 237, and wherein the purified inverse thermosensitive polymer has a polydispersity index of 1.5 to 1.0 and a molecular weight of between 5,000 and 35,000. Another aspect of the invention relates to use of such a composition in the preparation of a medicament for gastrointestinal mucosal resectioning according to any one of claims 7 to 10.

### Brief Description of the Figures

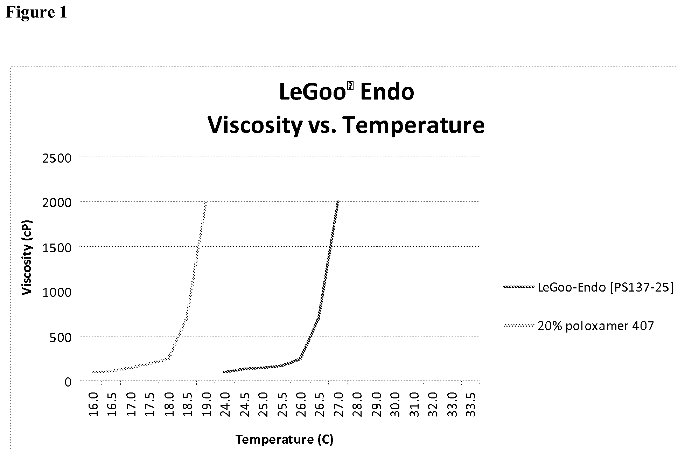

**Figure 1** depicts graphically the viscosities of LeGoo Endo (a 20-25% aqueous solution of purified poloxamer 237) and a 20% aqueous solution of unpurified poloxamer 407 as a function of temperature.

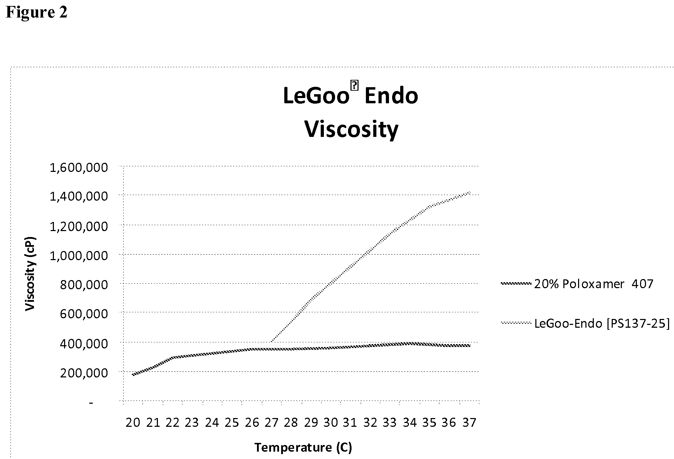

**Figure 2** depicts graphically the viscosities of LeGoo Endo (a 20-25% aqueous solution of purified poloxamer 237) and a 20% aqueous solution of unpurified poloxamer 407 as a function of temperature.

### Detailed Description of the Invention

Endoscopic mucosal resection (EMR) represents a major advance in minimally invasive surgery in the gastrointestinal tract. EMR is based on the concept that endoscopy provides visualization and access to the mucosa, the innermost lining of the gastrointestinal tract -- the site where most gastrointestinal cancers from the esophagus to the rectum have their origin. The method combines the therapeutic power of endoscopic surgery with the diagnostic power of pathology examination of resected tissue.

EMR first proliferated in Japan, where endoscopists were faced with a very high incidence of stomach cancer. This differs from most Western countries, including the United States, where colon cancer is a much more common disease. Most colon cancers arise in mucosal polyps, which project into the lumen of the colon, making them relatively easy to remove at endoscopy using wire loops to grasp the polyp base. The polyps are then excised with electric current, producing simultaneous cutting action and cauterization.

In contrast, in the stomach most cancers do not begin in polyps, but in only slightly elevated, flat, or slightly depressed mucosal dysplastic lesions. Such lesions are very difficult to grasp with a simple wire snare. Japanese endoscopists worked to develop a number of methods to elevate the diseased mucosal area so that snaring would be possible. Most of these techniques used fluid injection into the submucosa, the layer of the gastrointestinal tract immediately below the mucosa, to elevate the mucosa and allow it to be grasped with a snare. Unfortunately, the fluids used to date have not be optimal for the EMR because, for example, they are typically insufficiently viscous to provide a sufficiently durable, elevated surface. Were fluids available that were capable of providing the optimal durable, elevated mucosal surface, EMR could also be used effectively in the esophagus, where early cancer and premalignant dysplasia also tends to be nonpolypoid and flat, and also in the colon, where it can be used to assist in removal of both small and large flat or sessile polyps.

Unpurified inverse thermosensitive polymers could be considered for this indication, but unfortunately these polymers, which gel at body temperature, gel in the catheter used for injection because the catheter warms to body temperature soon after it is deployed within the colon or stomach.

Remarkably, a purified inverse thermosensitive polymer (LeGoo-endo ™), which displays a rapid reversible liquid to gel transition, has now been shown to be efficacious as a submucosal injection solution in *ex vivo* and *in vivo* porcine models of human endoscopic gastrointestinal mucosal resectioning (EMR). The rapid reversible liquid to gel transition achieved as a result its purified nature allows LeGoo-endo to be liquid at room temperature and to gel only as it emerges from the catheter at the EMR site. The mucosal elevation obtained with LeGoo-endo ™ was more durable than that obtained with other commonly used substances. Moreover, results with LeGoo-endo ™ were not subject to significant variations in terms of size and consistency. LeGoo-endo ™ performed well in *in vivo* colonic EMR. These results indicate that use of LeGoo-endo ™ or other purified inverse thermosensitive polymers may increase the safety and efficiency of human EMR procedures.

In certain embodiments, the bleb (i.e., the gel that provides the mucosal elevation) formed *in vivo* from the purified inverse thermosensitive polymer persists for about 30-180 minutes, about 45-150 minutes, about 60-120 minutes, about 75-100 minutes, about 90 minutes, about 80 minutes, about 70 minutes, about 60 minutes, about 50 minutes, about 40 minutes, or about 30 minutes.

In order to obtain the aforementioned results it was necessary to develop a method of injecting through a catheter into the colon or stomach a purified inverse thermosensitive polymer solution that transitions to a gel at body temperature. Among the challenges overcome was the fact that because the catheter quickly reaches body temperature while resident inside the body, the purified inverse thermosensitive polymer will gel inside the catheter prior to reaching the desired site for EMR. For example, due to gel formation in the catheter manual injection by pushing on the plunger of a syringe connected to a catheter is not workable, nor is injection assisted with a mechanical injector at pressures lower than 1200 psi; further, pressures higher than 1200 psi are precluded because they are sufficient to burst any conventional catheter. In other words, the method is not workable if the purified inverse thermosensitive polymer gels in the catheter (i.e., prior to reaching the EMR site), and pressures that can be tolerated by conventional catheters are insufficient to deliver in fluid form the purified inverse thermosensitive polymer to the EMR site.

Remarkably, the delivery problems were solved with a system comprising a high-pressure needle catheter connected to a syringe filled with purified inverse thermosensitive polymer, wherein said high-pressure needle catheter is contained within an administration device (e.g., a syringe pump) that generates pressure on the plunger of the syringe through a manual (e.g., screw), electrical or pressurized-gas mechanism. The higher pressures available and tolerated using said system have two functions: (a) pushing the viscous fluid through the catheter; and (b) allowing for a sufficiently rapid injection that purified inverse thermosensitive polymer entering the catheter from the room-temperature syringe constantly regulates the temperature of the catheter so that the polymer does not gel in the catheter during the residence time, and only gels after it emerges from the catheter and is brought into direct contact with the EMR site.

In sum, LeGoo-Endo -- denoted "PS137-25" in the Figures -- is an aqueous solution of purified poloxamer 237 with a viscosity at body temperature 3.9 times that of unpurified 20% poloxamer 407, allowing for the formation of a lasting bleb capable of withstanding the pressure exercised by the elastic mucosal membrane; moreover, the viscosities of LeGoo-Endo at 25 C and room temperature are less than a tenth of the viscosities of unpurified 20% poloxamer 407 at those temperatures, respectively, thereby preventing gel formation within the catheter during administration of LeGoo-Endo, in part because the catheter is continuously cooled by new LeGoo-Endo as it enters the catheter under pressure.

### Inverse Thermosensitive Polymers

In general, the inverse thermosensitive polymers used in the compositions of the invention, which become a gel at or about body temperature, can be injected into the patient's body in a liquid or soft gel form. The injected material once reaching body temperature undergoes a transition from a liquid or soft gel to a hard gel. The inverse thermosensitive polymers used in connection with the invention comprise a block copolymer with inverse thermal gelation properties. In general, biocompatible, biodegradable block copolymers that exist as a gel at body temperature and a liquid at below body temperature may also be used according to the present invention. Also, the inverse thermosensitive polymer can include a therapeutic agent, such as anti-angiogenic agents, hormones, anesthetics, antimicrobial agents (antibacterial, antifungal, antiviral), anti-inflammatory agents, diagnostic agents, or wound healing agents. Similarly, low concentrations of dye (such as methylene blue) or fillers can be added to the inverse thermosensitive polymer.

The molecular weight of the inverse thermosensitive polymer is between 5,000 and 35,000. Typically the polymer is in an aqueous solution. For example, typical aqueous solutions contain about 5% to about 30% polymer, or about 10% to about 25%. The molecular weight of a suitable inverse thermosensitive polymer may be, for example, between 7,000 and 20,000.

The pH of the inverse thermosensitive polymer formulation administered to the mammal is, generally, about 6.0 to about 7.8, which are suitable pH levels for injection into the mammalian body. The pH level may be adjusted by any suitable acid or base, such as hydrochloric acid or sodium hydroxide.

### Poloxamers (Pluronics)

Notably, Pluronic^{®} polymers have unique surfactant abilities and extremely low toxicity and immunogenic responses. These products have low acute oral and dermal toxicity and low potential for causing irritation or sensitization, and the general chronic and sub-chronic toxicity is low. In fact, Pluronic^{®} polymers are among a small number of surfactants that have been approved by the FDA for direct use in medical applications and as food additives (BASF (1990) Pluronic^{®} & Tetronic^{®} Surfactants, BASF Co., Mount Olive, N.J.). Recently, several Pluronic^{®} polymers have been found to enhance the therapeutic effect of drugs, and the gene transfer efficiency mediated by adenovirus. (March K L, Madison J E, Trapnell B C. "Pharmacokinetics of adenoviral vector-mediated gene delivery to vascular smooth muscle cells: modulation by poloxamer 407 and implication for cardiovascular gene therapy" Hum Gene Therapy 1995, 6, 41-53).

Poloxamers (or Pluronics), as nonionic surfactants, are widely used in diverse industrial applications. (Nonionic Surfactants: polyoxyalkylene block copolymers, Vol. 60. Nace VM, Dekker M (editors), New York, 1996. 280 pp.) Their surfactant properties have been useful in detergency, dispersion, stabilization, foaming, and emulsification. (Cabana A, Abdellatif AK, Juhasz J. "Study of the gelation process of polyethylene oxide. polypropylene oxide-polyethylene oxide copolymer (poloxamer 407) aqueous solutions." Journal of Colloid and Interface Science. 1997; 190: 307-312.) Certain poloxamines, e.g., poloxamine 1307 and 1107, also display inverse thermosensitivity.

Some of these polymers have been considered for various cardiovascular applications, as well as in sickle cell anemia. (Maynard C, Swenson R, Paris JA, Martin JS, Hallstrom AP, Cerqueira MD, Weaver WD. Randomized, controlled trial of RheothRx (poloxamer 188) in patients with suspected acute myocardial infarction. RheothRx in Myocardial Infarction Study Group. Am Heart J. 1998 May; 135 (5 Pt 1): 797-804; O'Keefe JH, Grines CL, DeWood MA, Schaer GL, Browne K, Magorien RD, Kalbfleisch JM, Fletcher WO Jr, Bateman TM, Gibbons RJ. Poloxamer-188 as an adjunct to primary percutaneous transluminal coronary angioplasty for acute myocardial infarction. Am J Cardiol. 1996 Oct 1;78(7):747-750; and Orringer EP, Casella JF, Ataga KI, Koshy M, Adams-Graves P, Luchtman-Jones L, Wun T, Watanabe M, Shafer F, Kutlar A, Abboud M, Steinberg M, Adler B, Swerdlow P, Terregino C, Saccente S, Files B, Ballas S, Brown R, Wojtowicz-Praga S, Grindel JM. Purified poloxamer 188 for treatment of acute vasoocclusive crisis of sickle cell disease: A randomized controlled trial. JAMA. 2001 Nov 7;286 (17):2099-2106. Also, see WO 2005/046438.)

Importantly, several members of this class of polymer, e.g., poloxamer 188, poloxamer 407, poloxamer 338, poloxamines 1107 and 1307, show inverse thermosensitivity within the physiological temperature range. (Qiu Y, Park K. Environment-sensitive hydrogels for drug delivery. Adv Drug Deliv Rev. 2001 Dec 31;53(3):321-339; and Ron ES, Bromberg LE Temperature-responsive gels and thermogelling polymer matrices for protein and peptide delivery Adv Drug Deliv Rev. 1998 May 4;31(3):197- 221.) In other words, these polymers are members of a class that are soluble in aqueous solutions at low temperature, but gel at higher temperatures. Poloxamer 407 is a biocompatible polyoxypropylene-polyoxyethylene block copolymer having an average molecular weight of about 12,500 and a polyoxypropylene fraction of about 30%; poloxamer 188 has an average molecular weight of about 8400 and a polyoxypropylene fraction of about 20%; poloxamer 338 has an average molecular weight of about 14,600 and a polyoxypropylene fraction of about 20 %; poloxamine 1,107 has an average molecular weight of about 14,000, poloxamine 1307 has an average molecular weight of about 18,000. Polymers of this type are also referred to as reversibly gelling because their viscosity increases and decreases with an increase and decrease in temperature, respectively. Such reversibly gelling systems are useful wherever it is desirable to handle a material in a fluid state, but performance is preferably in a gelled or more viscous state. As noted above, certain poly(ethyleneoxide)/poly(propyleneoxide) block copolymers have these properties; they are available commercially as Pluronic® poloxamers and Tetronic ® poloxamines (BASF, Ludwigshafen, Germany) and generically known as poloxamers and poloxamines, respectively. See U.S. Pat. Nos. 4,188,373, 4,478,822 and 4,474,751.

The average molecular weights of the poloxamers range from about 1,000 to greater than 16,000 Daltons. Because the poloxamers are products of a sequential series of reactions, the molecular weights of the individual poloxamer molecules form a statistical distribution about the average molecular weight. In addition, commercially available poloxamers contain substantial amounts of poly(oxyethylene) homopolymer and poly(oxyethylene)/poly(oxypropylene diblock polymers. The relative amounts of these byproducts increase as the molecular weights of the component blocks of the poloxamer increase. Depending upon the manufacturer, these byproducts may constitute from about 15 to about 50% of the total mass of the polymer.

### Purification of Inverse Thermosensitive Polymers

The inverse thermosensitive polymers may be purified using a process for the fractionation of water-soluble polymers, comprising the steps of dissolving a known amount of the polymer in water, adding a soluble extraction salt to the polymer solution, maintaining the solution at a constant optimal temperature for a period of time adequate for two distinct phases to appear, and separating physically the phases. Additionally, the phase containing the polymer fraction of the preferred molecular weight may be diluted to the original volume with water, extraction salt may be added to achieve the original concentration, and the separation process repeated as needed until a polymer having a narrower molecular weight distribution than the starting material and optimal physical characteristics can be recovered.

The purified inverse thermosensitive polymer used in the present invention is poloxamer 237 having a polydispersity
index from about 1.5 to about 1.0. In certain embodiments, the poloxamer 237
has a polydispersity index from about 1.2 to about 1.0.

The aforementioned process consists of forming an aqueous two-phase system composed of the polymer and an appropriate salt in water. In such a system, a soluble salt can be added to a single phase polymer-water system to induce phase separation to yield a high salt, low polymer bottom phase, and a low salt, high polymer upper phase. Lower molecular weight polymers partition preferentially into the high salt, low polymer phase. Polymers that can be fractionated using this process include polyethers, glycols such as poly(ethylene glycol) and poly(ethylene oxide)s, polyoxyalkylene block copolymers, such as poloxamers, poloxamines, and polyoxypropylene/ polyoxybutylene copolymers, and other polyols, such as polyvinyl alcohol. The average molecular weight of these polymers may range from about 800 to greater than 100,000 Daltons. See U.S. Patent 6,761,824

. The aforementioned purification process inherently exploits the differences in size and polarity, and therefore solubility, among the poloxamer molecules, the poly(oxyethylene) homopolymer and the poly(oxyethylene)/poly(oxypropylene) diblock byproducts. The polar fraction of the poloxamer, which generally includes the lower molecular weight fraction and the byproducts, is removed allowing the higher molecular weight fraction of poloxamer to be recovered. The larger molecular weight purified poloxamer (an example of a purified inverse thermosensitive polymer) recovered by this method has physical characteristics substantially different from the starting material or commercially available poloxamer including a higher average molecular weight, lower polydispersity and a higher viscosity in aqueous solution.

Other purification methods may be used to achieve the desired outcome. For example, WO 92/16484 discloses the use of gel permeation chromatography to isolate a fraction of poloxamer 188 that exhibits beneficial biological effects, without causing potentially deleterious side effects. The copolymer thus obtained had a polydispersity index of 1.07 or less, and was substantially saturated. The potentially harmful side effects were shown to be associated with the low molecular weight, unsaturated portion of the polymer, while the medically beneficial effects resided in the uniform higher molecular weight material. Other similarly improved copolymers were obtained by purifying either the polyoxypropylene center block during synthesis of the copolymer, or the copolymer product itself (*e.g.,* U.S. Pat. No. 5,523,492 and U.S. Pat. No. 5,696,298).

Further, a supercritical fluid extraction technique has been used to fractionate a polyoxyalkylene block copolymer as disclosed in U.S. Pat. No. 5,567,859

. A purified fraction was obtained, which was composed of a fairly uniform polyoxyalkylene block copolymer having a polydispersity of less than 1.17. According to this method, the lower molecular weight fraction was removed in a stream of carbon dioxide maintained at a pressure of 2200 pounds per square inch (psi) and a temperature of 40 °C.

Additionally, U.S. Pat. No. 5,800,711 discloses a process for the fractionation of polyoxyalkylene block copolymers by the batchwise removal of low molecular weight species using a salt extraction and liquid phase separation technique. Poloxamer 407 and poloxamer 188 were fractionated by this method. In each case, a copolymer fraction was obtained which had a higher average molecular weight and a lower polydispersity index as compared to the starting material. However, the changes in polydispersity index were modest and analysis by gel permeation chromatography indicated that some low-molecular-weight material remained. The viscosity of aqueous solutions of the fractionated polymers was significantly greater than the viscosity of the commercially available polymers at temperatures between 10 °C and 37 °C, an important property for some medical and drug delivery applications. Nevertheless, some of the low molecular weight contaminants of these polymers are thought to cause deleterious side effects when used inside the body, making it especially important that they be removed in the fractionation process. As a consequence, polyoxyalkylene block copolymers fractionated by this process are not appropriate for all medical uses.

As mentioned above, the use of these polymers in larger concentrations in humans requires removal of lower molecular weight contaminants present in commercial preparations. As was demonstrated in US Patent 5,567,859 ( Examples 8 & 9), the lower molecular weight contaminants are mostly responsible for the toxic effects seen. In a clinical trial using unpurified poloxamer 188, an unacceptable level of transient renal dysfunction was found (Maynard C, Swenson R, Paris JA, Martin JS, Hallstrom AP, Cerqueira MD, Weaver WD. Randomized, controlled trial of RheothRx (poloxamer 188) in patients with suspected acute myocardial infarction. RheothRx in Myocardial Infarction Study Group. Am Heart J. 1998 May; 135(5 Pt 1):797-804), while another clinical trial using purified poloxamer 188 specifically mentioned that no renal dysfunction was found (Orringer EP, Casella JF, Ataga KI, Koshy M, Adams-Graves P, Luchtman-Jones L, Wun T, Watanabe M, Shafer F, Kutlar A, Abboud M, Steinberg M, Adler B, Swerdlow P, Terregino C, Saccente S, Files B, Ballas S, Brown R, Wojtowicz-Praga S, Grindel JM. Purified poloxamer 188 for treatment of acute vasoocclusive crisis of sickle cell disease: A randomized controlled trial. JAMA. 2001 Nov 7;286(17):2099-2106.) Therefore, it seems imperative to utilize only fractionated poloxamers and poloxamines in EMR applications like the ones envisioned here. Furthermore, fractionation of these thermosensitive polymers leads to improved gels with stronger mechanical resistance and due to the improved thermosensitivity requires less polymer to achieve gelation (See for example US patent No. 6,761,824 on a purification scheme and the resultant viscosities).

### Drug Delivery in Conjunction with EMR Using Purified Inverse Thermosensitive Polymers

Therapeutically effective use of many types of biologically active molecules has not been realized simply because methods are not available to effect delivery of therapeutically effective amounts of such substances into the particular cells of a patient for which treatment would provide therapeutic benefit. New ways of delivering drugs at the right time, in a controlled manner, with minimal side effects, and greater efficacy per dose are sought by the drug-delivery and pharmaceutical industries.

The reversibly gelling polymers used in EMR methods according to the invention have physico-chemical characteristics that make them suitable delivery vehicles for conventional small-molecule drugs, as well as new macromolecular (*e.g*., peptides) drugs or other therapeutic products. Therefore, the composition comprising the purified inverse thermosensitive polymer may further comprise a pharmaceutic agent selected to provide a pre-selected pharmaceutic effect. A pharmaceutic effect is one which seeks to treat the source or symptom of a disease or physical disorder. Pharmaceutics include those products subject to regulation under the FDA pharmaceutic guidelines, as well as consumer products. Importantly, the compositions used EMR methods of the invention are capable of solubilizing and releasing bioactive materials. Solubilization is expected to occur as a result of dissolution in the bulk aqueous phase or by incorporation of the solute in micelles created by the hydrophobic domains of the poloxamer. Release of the drug would occur through diffusion or network erosion mechanisms.

Those skilled in the art will appreciate that the compositions used in EMR methods according to the invention may simultaneously be utilized to deliver a wide variety of pharmaceutic and personal care applications. To prepare a pharmaceutic composition, an effective amount of pharmaceutically active agent(s), which imparts the desirable pharmaceutic effect is incorporated into the reversibly gelling composition used in EMR methods according to the invention. Preferably, the selected agent is water soluble, which will readily lend itself to a homogeneous dispersion throughout the reversibly gelling composition. It is also preferred that the agent(s) is non-reactive with the composition. For materials, which are not water soluble, it is also within the scope of the invention to disperse or suspend lipophilic material throughout the composition. Myriad bioactive materials may be delivered using the compositions of the present invention; the delivered bioactive material includes anesthetics, antimicrobial agents (antibacterial, antifungal, antiviral), anti-inflammatory agents, diagnostic agents, and wound healing agents.

Because the reversibly gelling compositions of the present invention are suited for application under a variety of physiological conditions, a wide variety of pharmaceutically active agents may be incorporated into and administered from the compositions. The pharmaceutic agent loaded into the polymer networks of the purified inverse thermosensitive polymer may be any substance having biological activity, including proteins, polypeptides, polynucleotides, nucleoproteins, polysaccharides, glycoproteins, lipoproteins, and synthetic and biologically engineered analogs thereof.

A vast number of therapeutic agents may be incorporated in the polymers used in the present invention. In general, therapeutic agents which may be administered via compositions of the invention include, without limitation: antiinfectives such as antibiotics and antiviral agents; analgesics and analgesic combinations; anorexics; antihelmintics; antiarthritics; antiasthmatic agents; anticonvulsants; antidepressants; antidiuretic agents; antidiarrheals; antihistamines; antiinflammatory agents; antimigraine preparations; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary, peripheral and cerebral; central nervous system stimulants; cough and cold preparations, including decongestants; hormones such as estradiol and other steroids, including corticosteroids; hypnotics; immunosuppressives; muscle relaxants; parasympatholytics; psychostimulants; sedatives; and tranquilizers; and naturally derived or genetically engineered proteins, polysaccharides, glycoproteins, or lipoproteins. Suitable pharmaceuticals for parenteral administration are well known as is exemplified by the Handbook on Injectable Drugs, 6th Edition, by Lawrence A. Trissel, American Society of Hospital Pharmacists, Bethesda, Md., 1990.

The pharmaceutically active compound may be any substance having biological activity, including proteins, polypeptides, polynucleotides, nucleoproteins, polysaccharides, glycoproteins, lipoproteins, and synthetic and biologically engineered analogs thereof. The term "protein" is art-recognized and for purposes of this invention also encompasses peptides. The proteins or peptides may be any biologically active protein or peptide, naturally occurring or synthetic.

Examples of proteins include antibodies, enzymes, growth hormone and growth hormone-releasing hormone, gonadotropin-releasing hormone, and its agonist and antagonist analogues, somatostatin and its analogues, gonadotropins such as luteinizing hormone and follicle-stimulating hormone, peptide T, thyrocalcitonin, parathyroid hormone, glucagon, vasopressin, oxytocin, angiotensin I and II, bradykinin, kallidin, adrenocorticotropic hormone, thyroid stimulating hormone, insulin, glucagon and the numerous analogues and congeners of the foregoing molecules. The pharmaceutical agents may be selected from insulin, antigens selected from the group consisting of MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, bordetela pertussis virus, vaccinia virus, adenovirus, canary pox, polio vaccine virus, plasmodium falciparum, bacillus calmette geurin (BCG), klebsiella pneumoniae, HIV envelop glycoproteins and cytokins and other agents selected from the group consisting of bovine somatropine (sometimes referred to as BST), estrogens, androgens, insulin growth factors (sometimes referred to as IGF), interleukin I, interleukin II and cytokins. Three such cytokins are interferon-β, interferon-y and tuftsin.

Examples of bacterial toxoids that may be incorporated in the compositions used in EMR methods according to the invention are tetanus, diphtheria, pseudomonas A, mycobaeterium tuberculosis. Examples of that may be incorporated in the compositions used in EMR methods according to the invention are HIV envelope glycoproteins, *e.g*., gp 120 or gp 160, for AIDS vaccines. Examples of anti-ulcer H2 receptor antagonists that may be included are ranitidine, cimetidine and famotidine, and other anti-ulcer drugs are omparazide, cesupride and misoprostol. An example of a hypoglycaemic agent is glizipide.

Classes of pharmaceutically active compounds which can be loaded into that may be incorporated in the compositions used in EMR methods according to the invention include, but are not limited to, anti-AIDS substances, anti-cancer substances, antibiotics, immunosuppressants (e.g*.*, cyclosporine) anti-viral substances, enzyme inhibitors, neurotoxins, opioids, hypnotics, antihistamines, lubricants tranquilizers, anti-convulsants, muscle relaxants and anti-Parkinson substances, anti-spasmodics and muscle contractants, miotics and anti-cholinergics, anti-glaucoma compounds, anti-parasite and/or anti-protozoal compounds, anti-hypertensives, analgesics, anti-pyretics and anti-inflammatory agents such as NSAIDs, local anesthetics, ophthalmics, prostaglandins, anti-depressants, anti-psychotic substances, anti-emetics, imaging agents, specific targeting agents, neurotransmitters, proteins, cell response modifiers, and vaccines.

Exemplary pharmaceutical agents considered to be particularly suitable for incorporation in the compositions used in EMR methods according to the invention include, but are not limited to imidazoles, such as miconazole, econazole, terconazole, saperconazole, itraconazole, metronidazole, fluconazole, ketoconazole, and clotrimazole, luteinizing-hormone-releasing hormone (LHRH) and its analogues, nonoxynol-9, a GnRH agonist or antagonist, natural or synthetic progestrin, such as selected progesterone, 17-hydroxyprogeterone derivatives such as medroxyprogesterone acetate, and 19-nortestosterone analogues such as norethindrone, natural or synthetic estrogens, conjugated estrogens, estradiol, estropipate, and ethinyl estradiol, bisphosphonates including etidronate, alendronate, tiludronate, resedronate, clodronate, and pamidronate, calcitonin, parathyroid hormones, carbonic anhydrase inhibitor such as felbamate and dorzolamide, a mast cell stabilizer such as xesterbergsterol-A, lodoxamine, and cromolyn, a prostaglandin inhibitor such as diclofenac and ketorolac, a steroid such as prednisolone, dexamethasone, fluromethylone, rimexolone, and lotepednol, an antihistamine such as antazoline, pheniramine, and histiminase, pilocarpine nitrate, a beta-blocker such as levobunolol and timolol maleate. As will be understood by those skilled in the art, two or more pharmaceutical agents may be combined for specific effects. The necessary amounts of active ingredient can be determined by simple experimentation.

By way of example only, any of a number of antibiotics and antimicrobials may be included in the purified inverse thermosensitive polymers used in methods according to the invention invention. Antimicrobial drugs preferred for inclusion in compositions used in EMR methods according to the invention include salts of lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole and amanfadine and the like.

By way of example only, in the case of anti-inflammation, non-steroidal anti-inflammatory agents (NSAIDS) may be incorporated in the compositions used in EMR methods according to the invention, such as propionic acid derivatives, acetic acid, fenamic acid derivatives, biphenylcarboxylic acid derivatives, oxicams, including but not limited to aspirin, acetaminophen, ibuprofen, naproxen, benoxaprofen, flurbiprofen, fenbufen, ketoprofen, indoprofen, pirprofen, carporfen, and bucloxic acid and the like.

### Exemplification

The invention now being generally described, it will be more readily understood by reference to the following examples, which are included merely for purposes of illustration of certain aspects and embodiments of the present invention, and are not intended to limit the invention.

### Ex Vivo

Gastric endoscopic mucosal resections (120) were performed in fresh *ex vivo* porcine stomachs using three different solutions: normal saline solution (n = 40); HPMC (n = 40); and LeGoo-endo ™ (n = 40). Each submucosal injection was performed by injecting 5 mL of solution by using a 10-mL syringe with a 25-gauge needle. After creating a visually adequate submucosal elevation, the needle was kept in position for a short time to block the puncture site and prevent premature escape of solution. The stomachs were placed on a thermal pad for assuring a constant temperature (35-37° C). In all cases, the height and size of the bleb and the duration of the submucosal elevation were measured. When the elevation lasted visible in place 120 minutes, the test was finished.

The height of initial mucosal elevations was higher with LeGoo-endo ™ (10.3 ± 2.2 mm) than with saline (8.3 ± 2.6 mm, p<0.01) and HPMC (9.05 ± 2.3 mm, p=ns). No significant differences were observed regarding the large diameter of the elevations between LeGoo-endo ™ (34.7 ± 4.4 mm) and saline (36.7 ± 4 mm) or HPMC (33.7±4 mm). All the submucosal elevations with LeGoo-endo ™ lasted more than 120 minutes and the time was longer than with saline (20.9 ± 11 min, p<0.01) and HPMC (89 ± 32 min, p<0.01). After 120 minutes in place, the elevations performed with LeGoo-endo ™ showed no differences in size, shape and consistency.

### In Vivo

Five EMR were performed in the colon of 2 pigs with LeGoo-endo ™ using a 23-gauge scletotherapy needle with a 5-mL syringe and a balloon dilator gun. LeGoo-endo ™ was kept on ice during the intervention. Saline containing syringes were also kept on ice to cool the catheter immediately before poloxamer injections. After creating a visually adequate submucosal elevation, it was assessed as "small", "medium" or "big". Then, an "en bloc" resection of the lesion was performed using a needle knife or a polypectomy snare. All procedures were recorded and pictures were taken. In all cases, the size of the resected specimen was measured and surfaces were assessed by histologic evaluation.

The five EMR were located in the sigma between 18 and 25 cm from the anus margin. The height of initial mucosal elevation was large in 2 cases, medium in 2 cases, and small in 1 case. After the injection of the polymer, no reposition was needed. The mean volume injected was 6 ± 2.5 mL (range, 3-10 mL) and the mean size of the specimen resected was 2.6 ±1.1 cm (range 0.9-4 cm). The mean time for the resection was 5 ± 2 minutes (range, 2-8 minutes). During the resection, a large amount of gel was observed between the submucosa and the mucosa. No thermal injury was observed in the serosa surface and no perforations were reported. No changes were needed to electrocautery settings. In one case, a temporary bleeding from a submucosal vessel was observed. Histologic examination showed that submucosa layer was present in all the specimens.

## Claims

1. A composition comprising a purified inverse thermosensitive polymer for use in an endoscopic procedure for gastrointestinal mucosal resectioning in a mammal, wherein said purified inverse thermosensitive polymer is poloxamer 237, and wherein the purified inverse thermosensitive polymer has a polydispersity index of 1.5 to 1.0 and a molecular weight of between 5,000 and 35,000.

2. The composition for use of claim 1, wherein said composition has a transition temperature between about 10°C and about 40°C.

3. The composition for use of claim 1, wherein the volume of said composition at physiological temperature is about 80% to about 120% of its volume below its transition temperature.

4. The composition for use of claim 1, wherein said composition comprises about 5% to about 35% of said purified inverse thermosensitive polymer.

5. The composition for use of claim 1, wherein said purified inverse thermosensitive polymer has a polydispersity index from about 1.2 to about 1.0.

6. The composition for use of claim 1, wherein said composition further comprises a contrast-enhancing agent, optionally selected from the group consisting of radiopaque materials, paramagnetic materials, heavy atoms, transition metals, lanthanides, actinides, dyes, and radionuclide-containing materials.

7. Use of a composition in the preparation of a medicament for gastrointestinal mucosal resectioning, wherein the resectioning comprises administering submucosally to a region of a gastrointestinal mucosa in a mammal an effective amount of the composition comprising a purified inverse thermosensitive polymer, and surgically resecting said region of gastrointestinal mucosa, wherein said purified inverse thermosensitive polymer is poloxamer 237, and wherein the purified inverse thermosensitive polymer has a polydispersity index of 1.5 to 1.0 and a molecular weight of between 5,000 and 35,000.

8. The use of claim 7, wherein said composition has a transition temperature between about 10°C and about 40°C.

9. The use of claim 7, wherein the volume of said composition at physiological temperature is about 80% to about 120% of its volume below its transition temperature.

10. The use of claim 7, wherein said composition comprises about 5% to about 35% of said purified inverse thermosensitive polymer.

## Patentansprüche

1. Zusammensetzung, umfassend ein gereinigtes inverses thermoempfindliches Polymer, zur Verwendung in einem endoskopischen Verfahren für Magen-Darm-Schleimhaut-Resektion bei einem Säugetier, wobei das gereinigte inverse thermoempfindliche Polymer Poloxamer 237 ist und wobei das gereinigte inverse thermoempfindliche Polymer einen Polydispersitätsindex von 1,5 bis 1,0 und ein Molekulargewicht von zwischen 5.000 und 35.000 aufweist.

2. Zusammensetzung zur Verwendung nach Anspruch 1, wobei eine Übergangstemperatur der Zusammensetzung zwischen etwa 10 °C und etwa 40 °C liegt.

3. Zusammensetzung zur Verwendung nach Anspruch 1, wobei das Volumen der Zusammensetzung bei Körpertemperatur etwa 80 % bis etwa 120 % ihres Volumens unter ihrer Übergangstemperatur beträgt.

4. Zusammensetzung zur Verwendung nach Anspruch 1, wobei die Zusammensetzung etwa 5 % bis etwa 35 % des gereinigten inversen thermoempfindlichen Polymers umfasst.

5. Zusammensetzung zur Verwendung nach Anspruch 1, wobei das gereinigte inverse thermoempfindliche Polymer einen Polydispersitätsindex von etwa 1,2 bis etwa 1,0 aufweist.

6. Zusammensetzung zur Verwendung nach Anspruch 1, wobei die Zusammensetzung zudem ein kontrastverstärkendes Mittel umfasst, das gegebenenfalls aus der Gruppe bestehend aus für Röntgenstrahlen undurchlässigen Materialien, paramagnetischen Materialien, Schweratomen, Übergangsmetallen, Lanthanoiden, Actinoiden, Farbstoffen und Radionuklid enthaltenden Materialien ausgewählt ist.

7. Verwendung einer Zusammensetzung zur Herstellung eines Medikaments zur Magen-Darm-Schleimhaut-Resektion, wobei die Resektion die submucosale Verabreichung von einer wirksamen Menge der ein gereinigtes inverses thermoempfindliches Polymer umfassenden Zusammensetzung an eine Region einer Magen-Darm-Schleimhaut bei einem Säugetier und das chirurgische Resektionieren der Region der Magen-Darm-Schleimhaut umfasst, wobei das gereinigte thermoempfindliche Polymer Poloxamer 237 ist und wobei das gereinigte thermoempfindliche Polymer einen Polydispersitätsindex von 1,5 bis 1,0 und ein Molekulargewicht von zwischen 5.000 und 35.000 aufweist.

8. Verwendung nach Anspruch 7, wobei eine Übergangstemperatur der Zusammensetzung zwischen etwa 10 °C und etwa 40 °C liegt.

9. Verwendung nach Anspruch 7, wobei das Volumen der Zusammensetzung bei Körpertemperatur etwa 80 % bis etwa 120 % ihres Volumens unter ihrer Übergangstemperatur beträgt.

10. Verwendung nach Anspruch 7, wobei die Zusammensetzung etwa 5 % bis etwa 35 % des gereinigten inversen temperaturempfindlichen Polymers umfasst.

## Revendications

1. Composition comprenant un polymère thermosensible inverse purifié pour utilisation dans une intervention endoscopique de résection muqueuse gastro-intestinale chez un mammifère, où ledit polymère thermosensible inverse purifié est le poloxamère 237, et où le polymère thermosensible inverse purifié a un indice de polydispersité de 1,5 à 1,0 et une masse moléculaire comprise entre 5000 et 35 000.

2. Composition pour utilisation selon la revendication 1, où ladite composition a une température de transition comprise entre environ 10 °C et environ 40 °C.

3. Composition pour utilisation selon la revendication 1, où le volume de ladite composition à une température physiologique vaut environ 80 % à environ 120 % de son volume en dessous de sa température de transition.

4. Composition pour utilisation selon la revendication 1, où ladite composition comprend environ 5 % à environ 35 % dudit polymère thermosensible inverse purifié.

5. Composition pour utilisation selon la revendication 1, où ledit polymère thermosensible inverse purifié a un indice de polydispersité d'environ 1,2 à environ 1,0.

6. Composition pour utilisation selon la revendication 1, où ladite composition comprend en outre un agent amplifiant le contraste, éventuellement choisi dans le groupe constitué par les matériaux radio-opaques, les matériaux paramagnétiques, les atomes lourds, les métaux de transition, les lanthanides, les actinides, les colorants, et les matériaux contenant des radionucléides.

7. Utilisation d'une composition dans la préparation d'un médicament destiné à une résection muqueuse gastro-intestinale, où la résection comprend l'administration par voie sous-muqueuse à une région d'une muqueuse gastro-intestinale chez un mammifère d'une quantité efficace de la composition comprenant un polymère thermosensible inverse purifié, et la résection chirurgicale de ladite région de muqueuse gastro-intestinale, où ledit polymère thermosensible inverse purifié est le poloxamère 237, et où le polymère thermosensible inverse purifié a un indice de polydispersité de 1,5 à 1,0 et une masse moléculaire comprise entre 5000 et 35 000.

8. Utilisation selon la revendication 7, où ladite composition a une température de transition comprise entre environ 10 °C et environ 40 °C.

9. Utilisation selon la revendication 7, où le volume de ladite composition à une température physiologique vaut environ 80 % à environ 120 % de son volume en dessous de sa température de transition.

10. Utilisation selon la revendication 7, où ladite composition comprend environ 5 % à environ 35 % dudit polymère thermosensible inverse purifié.
